# EUROPÄISCHE PATENTSCHRIFT

(11) **EP 0 534 401 B1**
(45) Veröffentlichungstag und Bekanntmachung des Hinweises auf die Patenterteilung: **06.08.1997**
(21) Anmeldenummer: 92116278.0
(22) Anmeldetag: 23.09.1992
(51) Int. Cl.: A61N 1/05, A61F 2/02

(54) **Implantierbare medizinische Anordnung**
Implantable medical device
Appareil médical implantable

(30) Priorität: 25.09.1991 SE 9102778
(43) Veröffentlichungstag der Anmeldung: 31.03.1993
(73) Patentinhaber: Pacesetter AB, 171 95 Solna (SE)
(72) Erfinder: Lindegren, Ulf, S-122 35 Enskede (SE); Peterson, Lars-Olof, S-161 37 Bromma (SE); Stroetmann, Brigitte, W-8525 Uttenreuth (DE)
(74) Vertreter: Lettström, Richard Wilhelm

(56) Entgegenhaltungen:
- EP-A- 0 388 480
- WO-A-86/00795
- US-A- 4 281 668
- US-A- 4 281 669
- US-A- 4 336 811
- US-A- 4 711 251
- US-A- 4 784 161

## Beschreibung

Die Erfindung bezieht sich auf eine implantierbare medizinische Anordnung mit einer Oberfläche, die nach einer Implantation mit dem Gewebe eines Patienten in Verbindung steht, wobei mindestens ein Teil dieser Oberfläche mit mindestens einer Schicht aus einem Medikament versehen ist.

Eine Anordnung dieser Art ist durch die US-PS 4 711 251 bekannt. Die Anordnung, die in dieser Schrift eine Herzschrittmacherelektrode ist, weist auf ihrem Elektrodenkopf eine Medikamentschicht auf, die entzündungshemmend wirkt, wenn der Elektrodenkopf gegen die Herzwand anliegt. Auf diese Weise kann das Entstehen fibrosen Gewebes im Bereich des Elektrodenkopfes vermieden bzw. verringert werden. Der Nachteil der Medikamentschicht ist, daß sie in der Implantationsphase in Verbindung mit Körperflüssigkeiten kommt und somit wenigstens teilweise aufgelöst wird, bevor der Elektrodenkopf die entgültige Position an der Herzwand erreicht.

Wo 86/00795 beschreibt eine Prothese, die mit einer Antibiotikaschicht versehen ist. Damit die Antibiotikaschicht am inerten, thermoplastischen Material der Prothese haftet, wird dieses Material zuerst mit einem kationischen oberflächenaktiven Stoff, an dem das Antibiotikum haften kann, behandelt. Keine vollständige Schicht aus Antibiotika wird gebildet. Die Moleküle des kationischen Stoffs, die keine Bindung zu einer Antibiotikaverbindung haben, können eine Thrombose verursachen. Um dies zu vermeiden, wird die Prothese mit der Aufschlämmung einer partikelförmigen, am kationischen Stoff haltenden Kationenaustauschverbindung, z. B. Sepharos, behandelt. Danach werden die Sepharos-Partikel von der Prothese weggewaschen, wodurch der nicht-antibiotikagebundene, kationische, oberflächenaktive Stoff entfernt wird.

In der US-PS 4 304 591 ist eine hydrophile Polymerschicht, die bei Implantationen verwendet wird und als Träger für u.a. Medikamente dient, beschrieben. Auch in diesem Fall kann sich das Medikament, wenn es in Verbindung mit Körperflüssigkeit kommt, unerwünscht von der Polymerschicht lösen.

In der EP 0 388 480 A1 ist eine Herzschrittmacherlektrode beschrieben, deren Elektrodenkopf mit einer Schicht aus einem hydrophilen Polymer versehen ist, in der ein entzündungshemmendes Steroid eingebettet ist. Auf diese Weise wird zwar vermieden, daß das eingebettete Medikament vorzeitig aufgelöst wird, aber die Abgabe dieses Steroids ist schwer zu steuern. Außerdem ist die Menge des Medikaments, das in die Schicht eingebettet werden kann, in der Regel zu klein. Bei Bedarf von verhältnismäßig großen Mengen eines Medikaments muß die Polymerschicht derart dick gemacht werden, daß der Elektrodenkopf nicht mehr klein gehalten werden kann.

Der Erfindung liegt die Aufgabe zugrunde, eine Anordnung der eingangs genannten Art zu schaffen, bei der die Abgabe eines Medikaments in gewünschter Menge einfach und sicher gesteuert werden kann.

Diese Aufgabe ist erfindungsgemäß dadurch gelöst, daß die Medikamentschicht von mindestens einer Schicht aus einem Ionenaustauschermaterial bedeckt ist. Die Ionenaustauschermaterialschicht dient nicht nur als Schutz für die Medikamentschicht bei einer Implantation, sondern das Medikament wird abhängig von der Stärke der Ionenaustauschermaterialschicht in einer im voraus bestimmten Weise durchgelassen und abgegeben.

In einer vorteilhaften Ausbildung der Erfindung wird vorgeschlagen, daß die Anordnung ein Herzstimulationsgerät oder ein Teil davon ist. Mit Herzstimulationsgerät ist ein Herzschrittmacher oder ein Defibrillationsgerät gemeint.

Die Anordnung kann auch eine Herzstimulationselektrode sein. Mit Herzstimulationselektrode ist jede Art von Herzschrittmacher- oder Defibrillationselektrode gemeint.

Eine vorteilhafte Weiterbildung der Erfindung ergibt sich daraus, daß die Herzstimulationselektrode eine Herzschrittmacherelektrode mit mindestens einer Stimulationsoberfläche ist, bei der nur die Stimulationsoberfläche mit mindestens einer Medikamentschicht versehen ist, die von mindestens einer Ionenaustauschermaterialschicht bedeckt ist. Bei diesen kleinen Stimulationsoberflächen ist es von besonderer Bedeutung, daß bei einer implantierten Elektrode Medikament in einer bestimmten Menge durch die Ionenaustauschermaterialschicht diffundiert, um damit zu verhindern, daß fibröses Gewebe im Bereich dieser Stimulationsoberfläche gebildet wird.

Im Hinblicke auf eine günstige Ausgestaltung der Anordnung empfiehlt es sich, daß die Stimulationsoberfläche aus einem mikroporösen Material gebildet ist. Ein solches Material kann Kohlenstoff oder Titannitrid mit einer mikroporösen Struktur sein. Hierdurch ist es möglich, einen extrem kleinen Elektrodenkopf zu erhalten.

Die Erfindung kann vorteilhaft in Verbindung mit einer Herzschrittmacherelektrode verwendet werden, deren Elektrodenkopf eine Oberfläche von maximal 4 mm² aufweist. Bei einer derartig extrem kleinen Oberfläche kann nunmehr, ohne den Elektrodenkopf nennenswert zu vergrößern, eine ausreichende Menge Medikamente und eine deckende Schicht aus Ionenaustauschermaterial an dem Elektrodenkopf appliziert und der Herzwand zugeführt werden, um Entzündungen im Bereich des Kopfes zu vermeiden.

In einer weiteren Ausgestaltung der Erfindung wird vorgeschlagen, daß Medikamentschichten und Ionenaustauschermaterialschichten abwechselnd in mehreren Lagen an der Oberfläche angebracht sind. So kann eine länger andauernde Abgabe von Medikamenten an das Gewebe eines Patienten erreicht werden. Außerdem können Medikamente verschiedener Arten zwischen den Ionenaustauschermaterialschichten appliziert werden.

Eine weitere günstige Ausgestaltung der Erfindung ergibt sich, wenn die Stärke der Ionenaustauschermaterialschicht und die Stärke der Medikamentschicht unabhängig voneinander variiert werden können. Hierdurch kann die Steuerung der Abgabe der Medikamentmenge verfeinert werden.

Weitere Vorteile und Einzelheiten der Erfindung ergeben sich aus den Unteransprüchen.

Die Erfindung ist nachfolgend anhand eines in den Zeichnungen dargestellten Ausführungsbeispiels näher erläutert. Es zeigen:

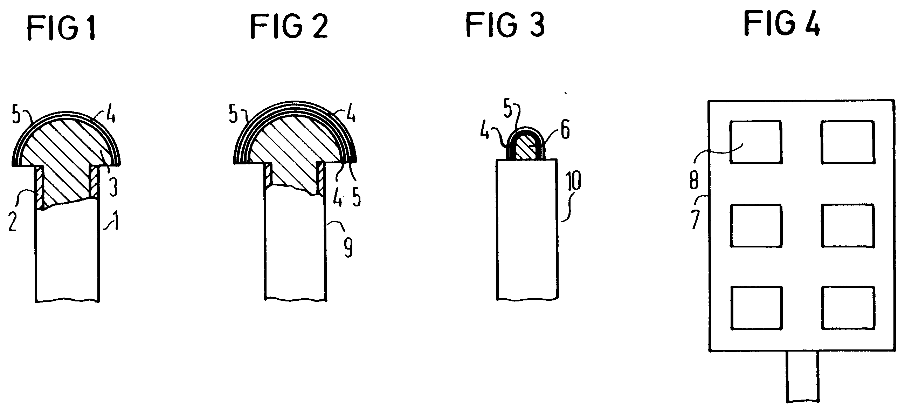

FIG 1 bis 4 Seitenansichten der Anordnung nach der Erfindung in verschiedenen Ausführungen.

In der FIG 1 ist das distale Ende einer Herzschrittmacherelektrode 1 zum Teil im Querschnitt abgebildet. Die Elektrode 1 weist eine Isolierung 2 und einen Elektrodenkopf 3 auf, der Stimulationsimpulse an das Herz eines Patienten überträgt. Die Stimulationsoberfläche des Elektrodenkopfes 3, die aus einem mikroporösen Material gebildet ist, ist mit einer Schicht 4 aus einem Medikament versehen, die mit einer Schicht 5 aus einem hydrophilen Ionenaustauschermaterial bedeckt ist. Die Ionenaustauschermaterialschicht 5 dient als Schutz für die Medikamentschicht 4 bei der Lagerung der Elektrode und in der Implantationsphase. Wenn die Herzschrittmacherelektrode 1 gegen die Herzwand appliziert ist, diffundiert das Medikament durch das Ionenaustausermaterial und erreicht die Herzwand und hemmt eine eventuelle Gewebereaktion, die sonst häufig zu einer Entzündung des Gewebes im Bereich des Elektrodenkopfes 3 führt. Die Stärken der Medikament- und Ionenaustauschermaterialschichten 4 und 5 sind zwischen 0,5 µm und 4 µm, vorzugsweise 2 µm. Die Stärken der genannten Schichten 4,5 können auch unabhängig voneinander variiert werden. Durch Abdecken der Medikamentschicht bzw. Schichten mit einer einzigen Ionenaustauschermaterialschicht diffundiert das Medikament in gewünschter Weise schnell durch die Ionenaustauschermaterialschicht 5. Werden mehrere Ionenaustauschermaterialschichten aufgebracht, geht der Durchlass des Medikaments langsammer. Es ist also möglich, durch die Stärke der Ionenaustauschermaterialschicht die Abgabegeschwindigkeit des Medikaments zu definieren. Durch Auflegen mehrerer Medkamentenschichten auf die Stimulationsoberfläche der Elektrode 1 kann eine verhältnismäßig große Menge von Medikamenten auf dem Elektrodenkopf 3 gelagert werden.

In der FIG 2 ist eine Herzschrittmacherelektrode 9 gezeigt, die der in der FIG 1 dargestellten Elektrode 1 ähnelt. In dieser FIG 2 ist dargestellt, daß die Medikamentschicht 4 und die Ionenaustauschermaterialschicht 5 abwechselnd in mehreren Lagen an der Stimulationsoberfläche angebracht sind. Durch diesen Aufbau wird eine länger andauernde Abgabe vom Medikament erhalten. Es ist auch möglich, verschiedene Sorten von Medikamenten zwischen den Ionenaustauschermaterialschichten zu verwenden, die sonst bei einer Mischung der Medikamentsorten schwer zu dosieren sein können.

In der FIG 3 ist eine Herzschrittmacherelektrode 10 mit einen Elektrodenkopf 6 gezeigt, dessen Oberfläche maximal 4 mm² ist. Auch ein derartig kleiner Elektrodenkopf kann mit Hilfe der Ionenaustauschermaterialschicht 5, die eine Medikamentschicht 4 deckt, eine verhälnismäßig große Menge Medikamente lagern, die an der Applikationsstelle des Elektrodenkopfes abgegen werden können. Der Kopf ist dabei trotz der aufgelegten Schichten 4,5 klein geblieben.

In der FIG 4 ist eine Defibrillationselektrode vom sog. Patch-Typ mit einer Anzahl Elektrodenplatten 8 gezeigt, die Stimulationsimpulse an das Herz überträgt. Bei einer derartigen Defibrillationselektrode können die Oberflächen der Elektrodenplatten 8, die ganze Seite oder beide Seiten der Patch-Elektrode mit einer Medikamentschicht, die mit einer Ionenaustauschermaterialschicht bedeckt ist, versehen sein. Auch eine hier nicht dargestellte endokardiale Defibrillationselektrode kann mit den erwähnten Schichten versehen werden.

Auch das Gehäuse eines Herzschrittmacher- bzw. Defibrillationsgerätes oder das Gehäuse einer Infusionspumpe kann ganz oder teilweise wie beschrieben mit der Medikament- und Ionenaustauschermaterialschichte versehen werden.

### Bezugszeichenliste

- 1,9,10: Herzschrittmacherelektrode, Herzstimulationselektrode
- 2: Isolierung
- 3,6: Elektrodenkopf
- 4: Medikamentschicht
- 5: Ionenaustauschermaterialschicht
- 7: Defibrillationselektrod, Herzstimulationselektrode
- 8: Elektrodenplatte

## Patentansprüche

1. Implantierbare medizinische Anordnung mit einer Oberfläche, die nach einer Implantation mit dem Gewebe eines Patienten in Verbindung steht, wobei mindestens ein Teil dieser Oberfläche mit mindestens einer Schicht aus einem Medikament versehen ist, **dadurch gekennzeichnet**, daß die Medikamentschicht (4) von mindestens einer Schicht (5) aus einem Ionenaustauschermaterial bedeckt ist.

2. Implantierbare medizinische Anordnung nach Anspruch 1, **dadurch gekennzeichnet**, daß die Anordnung ein Herzstimulationsgerät oder ein Teil davon ist.

3. Implantierbare medizinische Anordnung nach Anspruch 1 oder 2, **dadurch gekennzeichnet**, daß die Anordnung eine Herzstimulationselektrode (1,7,9,10) ist.

4. Implantierbare medizinische Anordnung nach Anspruch 3, **dadurch gekennzeichnet**, daß die Herzstimulationselektrode (1,7,9,10) eine Herzschrittmacherelektrode ist.

5. Implantierbare medizinische Anordnung nach Anspruch 4, **dadurch gekennzeichnet**, daß die Oberfläche aus einem mikroporösen Material gebildet ist.

6. Implantierbare medizinische Anordnung nach Anspruch 4 oder 5, **dadurch gekennzeichnet**, daß der Elektrodenkopf (6) der Herzschrittmacherelektrode (10) eine Oberfläche von maximal 4 mm² aufweist.

7. Implantierbare medizinische Anordnung nach Anspruch 1, **dadurch gekennzeichnet**, daß die Anordnung eine Infusionspumpe ist.

8. Implantierbare medizinische Anordnung nach einem der Ansprüche 4-6, **dadurch gekennzeichnet**, daß die Medikamentschichten (4) und Ionenaustauschermaterialschichten (5) abwechselnd in mehreren Lagen an der Oberfläche angebracht sind.

9. Implantierbare medizinische Anordnung nach einem der Ansprüche 1 bis 8, **dadurch gekennzeichnet**, daß die Stärke der Medikamentschicht oder Medikamentenschichten (4) zwischen 0,5 µm und 4 µm, vorzugsweise 2 µm ist.

10. Implantierbare medizinische Anordnung nach einem der Ansprüche 1 bis 9, **dadurch gekennzeichnet**, daß die Stärke der Schicht oder der Schichten des Ionenaustauschermaterials (5) zwischen 0,5 µm und 4 µm, vorzugsweise 2 µm ist.

## Claims

1. Implantable medical device having a surface which is in contact with the tissue of a patient after implantation, at least part of this surface being provided with at least a layer of a medicament, characterised in that the layer (4) of medicament is covered by at least a layer (5) of an ion exchanger material.

2. Implantable medical device according to claim 1, characterised in that the device is a cardiac stimulation device or a part thereof.

3. Implantable medical device according to claim 1 or 2, characterised in that the device is a cardiac stimulation electrode (1, 7, 9, 10).

4. Implantable medical device according to claim 3, characterised in that the cardiac stimulation electrode (1, 7, 9, 10) is a cardiac pacemaker electrode.

5. Implantable medical device according to claim 4, characterised in that the surface is formed by a microporous material.

6. Implantable medical device according to claim 4 or 5, characterised in that the electrode head (6) of the cardiac pacemaker electrode (10) has a surface area of a maximum of 4 mm².

7. Implantable medical device according to claim 1, characterised in that the device is an infusion pump.

8. Implantable medical device according to one of claims 4 to 6, characterised in that the layers (4) of medicament and the layers (5) of ion exchanger material are applied to the surface alternately in several layers.

9. Implantable medical device according to one of claims 1 to 8, characterised in that the thickness of the layer (4) of medicament or layers (4) of medicaments is between 0.5 µm and 4 µm, preferably 2 µm.

10. Implantable medical device according to one of claims 1 to 9, characterised in that the thickness of the layer (5) or layers (5) of ion exchanger material is between 0.5 µm and 4 µm, preferably 2 µm.

## Revendications

1. Dispositif médical implantable comportant une surface, qui, après implantation, est en liaison avec le tissu d'un patient, au moins une partie de cette surface étant munie d'au moins une couche constituée d'un médicament, caractérisé en ce que la couche (4) de médicament est recouverte d'au moins une couche (5) en une matière échangeuse d'ions.

2. Dispositif médical implantable suivant la revendication 1, caractérisé en ce que le dispositif est un appareil de stimulation cardiaque ou une partie de celui-ci.

3. Dispositif médical implantable suivant la revendication 1 ou 2, caractérisé en ce que le dispositif est une électrode (1,7,9,10) de stimulation cardiaque.

4. Dispositif médical implantable suivant la revendication 3, caractérisé en ce que l'électrode (1,7,9,10) de stimulation cardiaque est une électrode de stimulateur cardiaque.

5. Dispositif médical implantable suivant la revendication 4, caractérisé en ce que la surface est formée par un matériau microporeux.

6. Dispositif médical implantable suivant la revendication 4 ou 5, caractérisé en ce que la tête (6) d'électrode de l'électrode (10) de stimulateur cardiaque a une surface de 4mm² au plus.

7. Dispositif médical implantable suivant la revendication 1, caractérisé en ce que le dispositif est une pompe à perfusion.

8. Dispositif médical implantable suivant l'une des revendications 4 à 6, caractérisé en ce que les couches (4) de médicament et les couches (5) en une matière échangeuse d'ions sont montées en alternance en plusieurs couches à la surface.

9. Dispositif médical implantable suivant l'une des revendications 1 à 8, caractérisé en ce que l'épaisseur de la couche (4) de médicament ou des couches de médicament est comprise entre 0,5 µm et 4 µm, et est, de préférence, de 2µm.

10. Dispositif médical implantable suivit l'une des revendications 1 à 9, caracterisé en ce que l'épaisseur de la ou des couche(s) (5) en une matière échangeuse d'ions est comprise entre 0,5 µm et 4 µm, et est, de préférence, de 2 µm.
